# EUROPEAN PATENT APPLICATION

(11) **EP 4 656 660 A1**
(43) Date of publication of application: **03.12.2025**
(21) Application number: 24382584.1
(22) Date of filing: 31.05.2024
(51) Int. Cl.: C08B 37/16, A61K 31/724, A61K 47/40, C08L 5/16

(54) **ALFA-, BETA-, AND GAMMA- CYCLODEXTRIN DERIVATIVES DECORATED WITH (2-MERCAPTOETHYL)PHOSPHONIC ACID BRANCHES, SYNTHESIS AND APPLICATIONS**

(71) Applicant: Universidad de Almeria, 04120 La Cañada de San Urbano (Almeria) (ES)
(72) Inventor: LIROLA MANZANO, María de los Ángeles, 04120 La Cañada de San Urbano-Almería (ES); CASAS SOLVAS, Juan Manuel, 04120 La Cañada de San Urbano-Almería (ES); VARGAS BERENGUEL, Antonio, 04120 La Cañada de San Urbano-Almería (ES)
(74) Representative: TRBL Intellectual Property

(57) **Abstract**

The present invention refers to α-, β-, or γ-cyclodextrin derivatives wherein the hydroxyl groups at all C-6 positions of the α-, β-, or γ-cyclodextrin are replaced with 2-mercaptoethylphosphonic acid as (2-phosphonoethyl)thio branches. The invention further describes a method for obtaining α-, β-, or γ-cyclodextrin derivatives in accordance with the invention, wherein the method comprises: (a) preparing α-, β-, or γ-cyclodextrin derivatives where the hydroxyl groups at all C-6 positions of the α-, β-, or γ-cyclodextrin are replaced with iodine atoms; (b) replacing the iodine atoms at all C-6 positions of the α-, β-, or γ-cyclodextrin with [2-(diethoxyphosphoryl)ethyl]thio branches by using the isothiouronium salt of diethyl 2-mercaptoethylphosphonate; and (c) hydrolysing the diethyl esters of the [2-(diethoxyphosphoryl)ethyl]thio branches to give phosphonic acids as (2-phosphonoethyl)thio branches (-S-CH₂-CH₂-PO(OH)₂). Finally, the invention describes several uses of the α-, β-, or γ-cyclodextrin derivatives obtained in accordance with the invention.

## Description

### FIELD OF THE INVENTION

The present invention refers to structural analogues of Sualphadex, Subetadex and Sugammadex. Specifically, the invention refers to structural analogues of the latter compounds which derive from α-, β- and γ-cyclodextrin by perfunctionalization of the primary face of α-, β- and γ-cyclodextrin with (2-mercaptoethyl)phosphonic acid residues.

### BACKGROUND OF THE INVENTION

Cyclodextrins (IUPAC semi-systematic nomenclature: cyclomaltoses) are cyclic oligosaccharides composed of a number of glucose units bonded in a ring fashion. The three naturally occurring cyclodextrins present, respectively, six, seven, or eight α-D-glucose moieties linked through 1→4 glycosidic bonds. Spatial structure of cyclodextrins defines a hollow truncated cone shape, where the outer surface is hydrophilic and the inner side is slightly hydrophobic, which enables these compounds to encapsulate lipophilic substances in water by the formation of non-covalent inclusion complexes. These properties allow their use in a wide variety of applications.

In addition, cyclodextrins can undergo structural modifications leading to changes in their properties, particularly referring to their solubility and their ability to form inclusion complexes. In this regard, Sualphadex, Subetadex and Sugamadex are modified α-, β-, and γ-cyclodextrin derivatives in which the C-6 positions of the cyclodextrin are decorated with (2-carboxyethyl)thio branches. Such modification gives rise to characteristic properties for these compounds, which allow specific applications for them. Thus, Sugammadex is marketed under the name Bridion^{®} as an agonist of the muscle relaxant rocuronium, used as an anesthetic (Friedrich K. Pühringer et al., "Reversal of profound, high-dose rocuronium-induced neuromuscular blockade by sugammadex at two different time points: an international, multicenter, randomized, dose-finding, safety assessor-blinded, phase II trial", Clinical Trial Anesthesiology, 2008, 109, 188-197). Sugammadex is also used as an ampicillin carrier agent (Davide Maffeo et al., "Positive effect of natural and negatively charged cyclodextrins on the stabilization of penicillins towards β-lactamase degradation due to inclusion and external guest-host association. An NMR and MS study", Organic & Biomolecular Chemistry, 2006, 4, 1297-1304), or as packing material for HPLC columns in the form of Cu complexes (Zhentao Li et al., "γ-Cyclodextrin metal-organic framework supported by polydopamine as stationary phases for electrochromatographic enantioseparation", Talanta, 2020, 218, 121160). Moreover, subetadex is used as a carrier agent for anticancer drugs such as irinotecan, topotecan, doxorubicin, camptothecin, docetaxel, or idarubicin (Jian-Guang Cheng et al., "Selective binding and controlled release of anticancer drugs by polyanionic cyclodextrins", Bioorganic & Medicinal Chemistry, 2018, 26, 2287-2290). There are also common applications for all the three compounds (Sualphadex, Subetadex and Sugammadex) as chiral selectors in capillary electrophoresis (Minghui Tong et al., "A convenient and efficient 4-(diethylamino)-butylamine-labeled polarity-response-homodispersed strategy for absolute quantification of carboxyl submetabolome: Monitoring the whole progressive course of hepatocellular carcinoma", Journal of Chromatography A, 2022, 1683, 463506), or as drugs for the treatment or prevention of lysosomal diseases (WO2022/070092 A1).

In view of the interesting properties that can be obtained by the functionalization of α-, β- and γ-cyclodextrins, as clearly demonstrated in the case of compounds such as Sualphadex, Subetadex and Sugammadex, there is an interest in this technical field to provide alternatively modified α-, β- and γ-cyclodextrin derivatives that may improve the properties of those compounds already available, particularly suited for each specific application.

### BRIEF DESCRIPTION OF THE DRAWINGS

- Figure 1: It shows the structures of (A) α-cyclodextrin (cyclomaltohexaose); (B) β-cyclodextrin (cyclomaltoheptaose); and (C) γ-cyclodextrin (cyclomaltooctaose).
- Figure 2: It shows the structures of (A) 2-(2-(diethoxyphosphoryl)ethyl)isothiouronium bromide; (B) hexakis(6-deoxy-6-iodo)cyclomaltohexaose; (C) heptakis(6-deoxy-6-iodo)cyclomaltoheptaose; and (D) octakis(6-deoxy-6-iodo)cyclomaltooctaose.
- Figure 3: It shows the structures of (A) hexakis{6-deoxy-6-S-[2'-(diethoxyphosphoryl)ethyl]-6-thio}cyclomaltohexaose; (B) heptakis{6-deoxy-6-S-[2'-(diethoxyphosphoryl)ethyl]-6-thio}cyclomaltoheptaose; and (C) octakis{6-deoxy-6-S-[2'-(diethoxyphosphoryl)ethyl]-6-thiolcyclomaltooctaose.
- Figure 4: It shows the structures of (A) hexakis[6-deoxy-6-S-(2'-phosphonoethyl)-6-thio]cyclomaltohexaose; (B) heptakis[6-deoxy-6-S-(2'-phosphonoethyl)-6-thio]cyclomaltoheptaose; and (C) octakis[6-deoxy-6-S-(2'-phosphonoethyl)-6-thio]cyclomaltooctaose.
- Figure 5: It shows the structure of rocuronium bromide, specifically indicating the position of the 18-CH₃ and 19-CH₃ methyl groups.
- Figure 6: Partial ¹H NMR spectrum (600 MHz) of a mixture containing decreasing concentrations of rocuronium bromide [from 1 mM in a) to 0.4 mM in k)] and increasing concentrations of product 10 [from 0 mM in spectrum a) to 0.9 mM in spectrum k)] in 20 mM phosphate buffer, pH 7.2, in D₂O.
- Figure 7: Graphical plot of the complexation percentage of rocuronium bromide versus Sugammadex (black graph) and product 10 (gray graph) molar fractions.

### DETAILED DESCRIPTION OF THE INVENTION

The present invention provides a solution to the state-of-the-art requirements mentioned above. Specifically, the present invention describes a synthetic methodology allowing the efficient linkage of phosphonic acid groups to α-, β- and γ-cyclodextrin through well-defined spacers and stoichiometries, so as to resulting α-, β- and γ-cyclodextrin derivatives contain 2-mercaptoethylphosphonic acid residues as (2-phosphonoethyl)thio branches at all C-6 positions of their primary faces. The synthetic procedure consists of the steps of (i) iodination of all C-6 positions of the native cyclodextrins, replacing the OH groups at those positions with iodine atoms, (ii) replacement of the iodine atoms with [2-(diethoxyphosphoryl)ethyl]thio branches by using the isothiouronium salt of diethyl 2-mercaptoethylphosphonate, and (iii) hydrolysis of the diethyl esters to give free phosphonic acid groups.

The invention is more specifically defined by describing the following aspects:

In a first aspect the invention relates to α-, β-, or γ-cyclodextrin derivatives wherein the hydroxyl groups at all C-6 positions of the α-, β-, or γ-cyclodextrin are replaced with 2-mercaptoethylphosphonic acid residues as [2-(diethoxyphosphoryl)ethyl]thio branches according to formula 1: 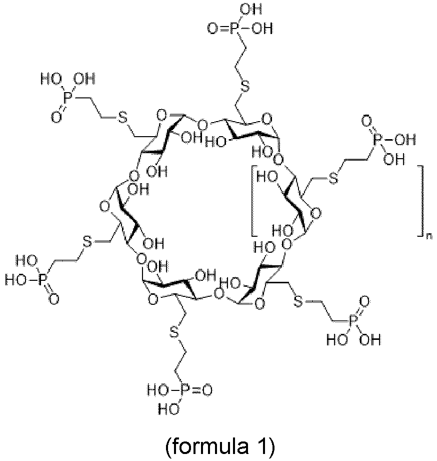 wherein n = 6-8.

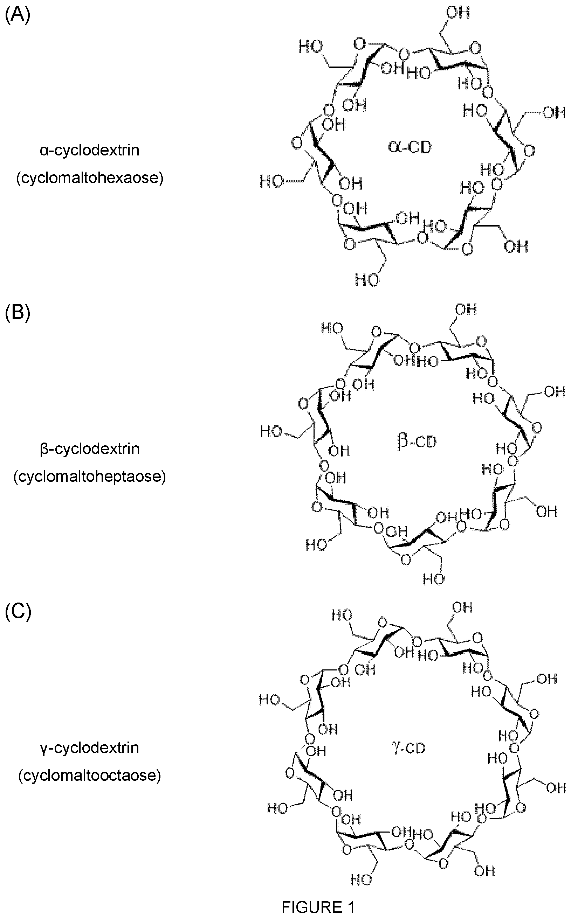

In the context of the present invention, the term "α-cyclodextrin" (Figure 1A) refers to a cyclic hexasaccharide derived from glucose with the semi-systematic IUPAC nomenclature cyclomaltohexaose and CAS number 10016-20-3 (hydrate). In the context of the present invention, the term "β-cyclodextrin" (Figure 1B) refers to a cyclic heptasaccharide derived from glucose with the semi-systematic IUPAC nomenclature cyclomaltoheptaose and CAS number 7585-39-9-9 (hydrate). In the context of the present invention, the term "γ-cyclodextrin" (Figure 1C) refers to a cyclic octasaccharide derived from glucose with the semi-systematic IUPAC nomenclature cyclomaltooctaose and CAS number 17465-86-0 (hydrate). α-, β-, and γ-cyclodextrin compounds consist of glucose monomers linked by α-(1→4) glycosidic bonds, where each glucose monomer has a hydroxyl group at its C-6 carbon. As described herein, 2-mercaptoethylphosphonic acid has the condensed formula: HS-CH₂-CH₂-P(O)(OH)₂. In particular embodiments of the present invention, the α-, β-, or γ-cyclodextrin derivative in accordance with the present invention is hexakis[6-deoxy-6-S-(2'-phosphonoethyl)-6-thio]cyclomaltohexaose, heptakis[6-deoxy-6-S-(2'-phosphonoethyl)-6-thio]cyclomaltoheptaose, or octakis[6-deoxy-6-S-(2'-phosphonoethyl)-6-thio]cyclomaltooctaose.

The α-, β-, or γ-cyclodextrin derivatives described within the invention are structural analogues of Sualphadex, Subetadex and Sugammadex, which are molecules based on α-, β- and γ-cyclodextrin scaffolds, respectively, decorated with 3-mercaptopropionic acid residues as (2-carboxyethyl)thio branches. The compounds described within the invention are analogous derivatives decorated with 2-mercaptoethylphosphonic acid residues as (2-phosphonoethyl)thio branches.

In another aspect, the invention relates to a method for obtaining α-, β-, or γ-cyclodextrin derivatives in accordance with the present invention, wherein the method comprises:
a) preparing α-, β-, or γ-cyclodextrin derivatives where the hydroxyl groups at all C-6 positions of the α-, β-, or γ-cyclodextrin are replaced with iodine atoms;
b) replacing the iodine atoms at all C-6 positions of the α-, β-, or γ-cyclodextrin with [2-(diethoxyphosphoryl)ethyl]thio branches by using the isothiouronium salt of diethyl 2-mercaptoethylphosphonate; and
c) hydrolysing the diethyl esters of the [2-(diethoxyphosphoryl)ethyl]thio branches to give phosphonic acids as (2-phosphonoethyl)thio branches (-S-CH₂-CH₂-PO(OH)₂).

In the context of the invention, a person skilled in the art understands that the first step of the method, of obtaining α-, β-, or γ-cyclodextrin derivatives where the hydroxyl groups at all C-6 positions of the α-, β-, or γ-cyclodextrin are replaced with iodine atoms, is known in the prior art (Baer et al., "Improved preparation of hexakis(6-deoxy) cyclomaltohexaose and heptakis(6-deoxy)cyclomaltoheptaose," Carbohydrate Research, 1992, 228, 307-314). A suitable method, in the context of the invention, for the preparation of α-, β-, or γ-cyclodextrin derivatives where the hydroxyl groups at all C-6 positions of the α-, β-, or γ-cyclodextrin are replaced with iodine atoms may comprise:
a) Adding I₂ to a solution of triphenylphosphine (Ph₃P) in dry *N,N*-dimethylformamide (DMF).
b) Adding α-, β-, or γ-cyclodextrin to the previous solution.
c) Stirring for 15-30 hours at 70-90°C in the lack of atmospheric moisture.
d) Alkalinizing with 3M NaOMe in MeOH at pH 9-10 at a temperature of 1-10°C for 10-60 minutes leading to the formate esters solvolysis.
e) Precipitating by the addition of MeOH or a mixture of water and ice.
f) Washing the solid with MeOH by using a Soxhlet apparatus, or redissolving the solid in DMF and reprecipitating with MeOH.

In the context of the invention, the second step of the method, of replacing the iodine atoms with [2-(diethoxyphosphoryl)ethyl]thio branches by using the isothiouronium salt of diethyl 2-mercaptoethylphosphonate, can be performed by means of a procedure comprising:
a) Stirring a solution of the iodinated α-, β-, or γ-cyclodextrin derivative, 2-(2-(diethoxyphosphoryl)ethyl)isothiouronium bromide, and cesium carbonate in dry DMF.
b) Purifying the phosphonated product, preferably by column chromatography using acetonitrile-water as eluent.

In embodiments of the present invention, the iodinated α-, β-, or γ-cyclodextrin derivative can be selected from hexakis(6-deoxy-6-iodo)cyclomaltohexaose, heptakis(6-deoxy-6-iodo)cyclomaltoheptaose and octakis(6-deoxy-6-iodo)cyclomaltooctaose, depending respectively on whether the cyclodextrin scaffold is composed of 6, 7 or 8 glucose monomers.

In the context of the invention, the third step of the method, of hydrolysing diethyl phosphonic esters on the [2-(diethoxyphosphoryl)ethyl]thio brances to give phosphonic acid groups as a (2-phosphonoethyl)thio (-S-CH₂-CH₂-CH₂-PO(OH)₂) branches, can be performed by means of a procedure comprising:
a) Adding trimethylsilyl bromide to a solution of hexakis{6-deoxy-6-S-[2'-(diethoxyphosphoryl)ethyl]-6-thio}cyclomaltohexaose, heptakis{6-deoxy-6-S-[2'-(diethoxyphosphoryl)ethyl]-6-thio}cyclomaltoheptaose or octakis{6-deoxy-6-S-[2'-(diethoxyphosphoryl)ethyl]-6-thio}cyclomaltooctaose, depending respectively on whether the cyclodextrin scaffold is composed of 6, 7 or 8 glucose monomers, in dry DMF at 0 °C under N₂ atmosphere, and stirring at room temperature overnight (i. e., 8-16 hours).
b) Adding water, evaporating the solvent and suspending the residue in MeCN to obtain a precipitate.

In particular embodiments, the method can include steps for filtering and washing the resulting precipitate, in which the method further comprises:
c) Filtering the precipitate and washing with MeCN;
d) Dissolving the precipitate in water; and
e) Freeze-drying to produce the final product.

The compounds synthesized in the context of the present invention are structural analogues of Sugammadex (Bridion^{®}) and its smaller analogues Subetadex and Sualphadex, and have potentially the same applications (reversal of rocuronium and vecuronium anaesthetics, chiral selectors in capillary electrophoresis, drugs against lysosomal diseases, solubilizing agents of anticancer drugs).

Therefore, in one aspect the invention relates to an α-, β-, or γ-cyclodextrin derivate in accordance with the present invention for use in medicine. In additional aspects, the invention relates to an α-, β-, or γ-cyclodextrin derivate in accordance with the present invention for use in the treatment or prevention of lysosomal diseases, or for use in the antagonistic treatment of the anaesthetics rocuronium and vecuronium. Additionally, the invention considers the use of an α-, β-, or γ-cyclodextrin derivate according to the present invention as a carrier of ampicillin, or as a carrier of anticancer drugs.

Finally, the invention relates in a last aspect to the use of an α-, β-, or γ-cyclodextrin derivate in accordance with the present invention as packing material for HPLC columns, preferably in the form of Cu complexes, or as a chiral selector in capillary electrophoresis.

All of the terms and embodiments described above are applicable to any aspect and embodiment of the invention. In accordance with the present invention, the singular terms "the", "this", "that", "one", "a", refer equally to its corresponding plural terms "these", "those", "ones", "some", unless it is clear from the context that clearly the term refers to a species in the singular. The term "comprises" or "comprising" as used herein also describes "consists of" or "consisting of' in accordance with generally accepted patent practice.

Any of the parametric ranges of time (t), temperature (T), etc. described herein may be used, in different embodiments of the invention, in isolation or in combination.

### EXAMPLES

The present invention is described by means of the following examples, which are to be considered as merely illustrative and not limiting the scope of the invention.

### Eiemple 1: Synthesis of 2-(2-(diethoxyphosphoryl)ethyl)isothiouronium bromide

This example describes the synthesis of 2-(2-(diethoxyphosphoryl)ethyl)isothiouronium bromide, where 2-(2-(diethoxyphosphoryl)ethyl)isothiouronium bromide is a common reagent for the preparation of all compounds of the present invention.

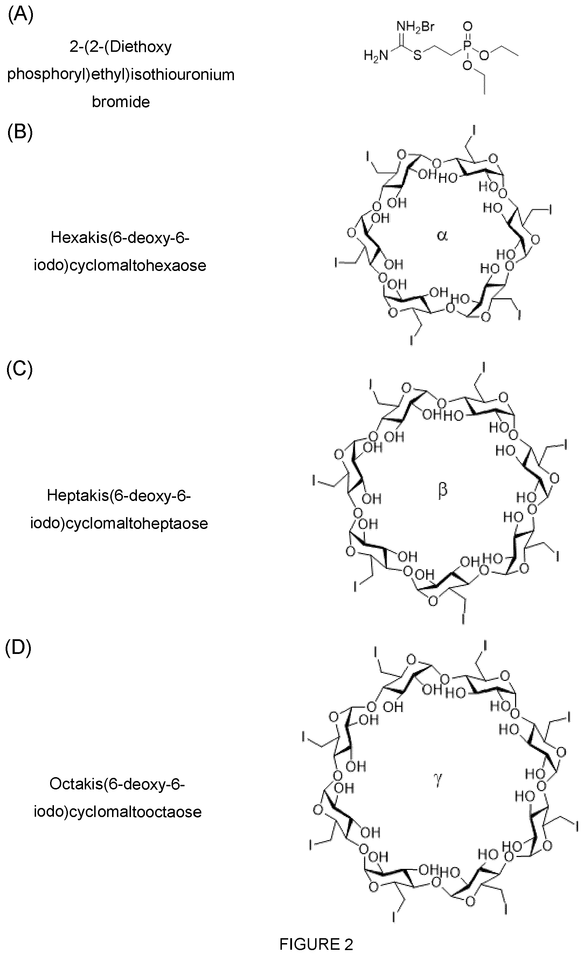

Preparation of reagent 1, 2-(2-(diethoxyphosphoryl)ethyl)isothiouronium bromide (Figure 2A): This compound was prepared following the method described by Schapman et al. ("Ignifugation de polyuréthanes: I. Utilisation d'un polyol insaturé modifié par le 1-thioéthyl diéthylphosphonate", European Polymer Journal, 2000, 36, 1865-1873). Diethyl 2-bromoethylphosphonate (1 g, 740 µL, 4.081 mmol) was added to a solution of thiourea (0.342 g, 4.489 mmol) in dry THF under inert atmosphere, and the resulting mixture was refluxed overnight. The mixture was filtered at 45 °C, and the residue was washed with warm tetrahydrofuran (THF) (45 °C) and dried under vacuum at 60 °C to afford 2-(2-(diethoxyphosphoryl)ethyl)isothiouronium bromide [reagent 1] (1.210 g, 3.767 mmol, 91 %) as a white solid. This white solid is characterized by ¹H-NMR (300 MHz, DMSO-d₆), δ (ppm): 9.11 (bs, 4H, NH₂, NH₂Br), 4.07-3.98 (m, 4H, OCH₂), 3.35-3.26 (m, 2H, SCH₂), 2.23-2.14 (m, 2H, CH₂P), 1.24 (t, 6H, ³*J* = 7.0 Hz, CH₃).

### Eiemple 2: Synthesis of hexakis[6-deoxy-6-S-(2'-phosphonoethyl)-6-thio]cyclomaltohexaose

This example describes the synthesis of hexakis[6-deoxy-6-S-(2'-phosphonoethyl)-6-thio]cyclomaltohexaose, analogue of Sualphadex with (2-phosphonoethyl)thio branches instead of (2-carboxyethyl)thio branches, based on an α-cyclodextrin core.

Preparation of reagent 2, hexakis(6-deoxy-6-iodo)cyclomaltohexaose (Figure 2B): Hexakis(6-deoxy-6-iodo)cyclomaltohexaose was prepared by the procedure described by Baer et al. ("Improved preparation of hexakis(6-deoxy)cyclomaltohexaose and heptakis (6-deoxy) cyclomaltoheptaose", Carbohydrate Research, 1992, 228, 307-314). Briefly, heptakis(6-deoxy-6-iodo)cyclomaltohexaose was prepared by the following procedure:

I₂ (2.8 g) was added in small portions to a stirred solution of triphenylphosphine (Ph₃P) (2.8 g) in dry *N,N*-dimethylformamide (DMF) (10 mL). After 30 min, α-cyclodextrin (0.5 g, dried under vacuum at 50 °C in the presence of P₂O₅ until constant weight) was also added. The mixture was stirred for 18 h at 80 °C under conditions preventing atmospheric moisture, then concentrated to half volume under reduced pressure, and cooled to 5 °C. It was then alkalinized with 3M NaOMe in MeOH to pH 9-10, and kept at room temperature for 30 min to allow formate esters solvolysis. The solution was then poured into a mixture of water (100 mL) and ice, and the solid was filtered under vacuum and washed with MeOH (100 mL) and CH₂Cl₂ (100 mL). The residue was redissolved in DMF (17 mL) and reprecipitated by addition of MeOH (40 mL), filtered again and washed with MeOH (40 mL). The resulting dark yellow solid was dried under vacuum at 60 °C until constant weight (0.7 g, 79%). The spectroscopic data for this material agreed with those described by Baer et al. (*supra*).

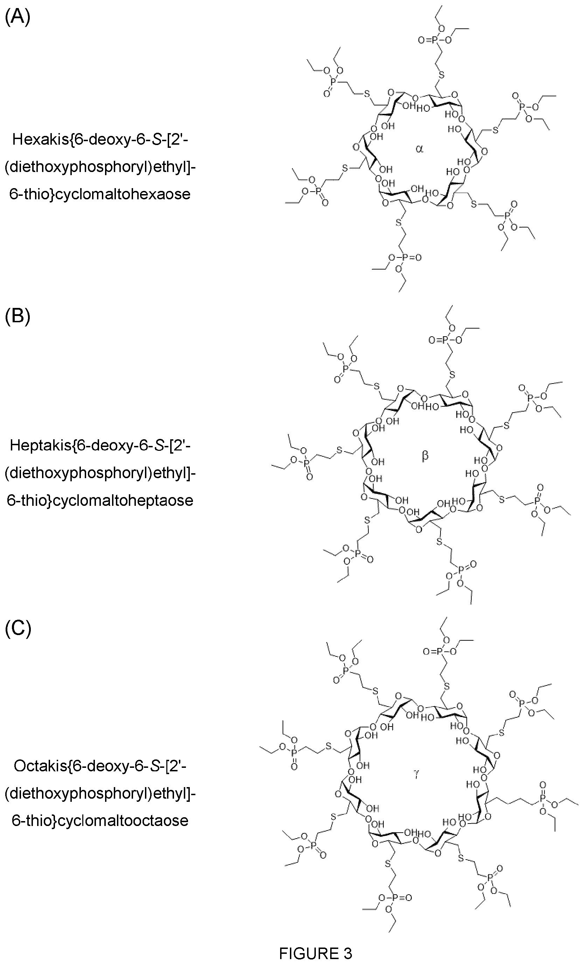

Preparation of product 3, hexakis{6-deoxy-6-S-[2'-(diethoxyphosphoryl)ethyl]-6-thiolcyclomaltohexaose (Figure 3A): A solution of heptakis(6-deoxy-6-iodo)cyclomaltohexaose [reagent 2] (0.100 g, 0.061 mmol), 2-(2-(diethoxyphosphoryl)ethyl)isothiouronium bromide [reagent 1] (0.231 g, 0.719 mmol) and cesium carbonate (0.360 g, 1.105 mmol) in dry DMF (5 mL) was stirred overnight. The solvent was then evaporated under vacuum and the residue was purified by column chromatography using acetonitrile-water (MeCN-H₂O 10:3) as eluent to afford hexakis{6-deoxy-6-S-[2'-(diethoxyphosphoryl)ethyl]-6-thio}cyclomaltohexaose [product 3] (0.093 g, 0.045 mmol, 74 %) as a white solid. ¹H NMR (600 MHz, DMSO-d₆) δ (ppm) 5.84 (d, 6H, ³*J* = 6.9 Hz, OH-2), 5.71 (d, 6H, ³*J* = 2.2 Hz, OH-3), 4.85 (d, 6H, ³*J*_{1*,*2} = 3.1 Hz, H-1), 4.04-3.94 (m, 24H, OC*H*₂CH₃), 3.85 (dt, 6H, *J* = 8.1 Hz, *J* = 2.4 Hz, H-5), 3.71 (dt, 6H, *J* = 9.4 Hz, *J* = 2 Hz, H-3), 3.44 (t, 6H, *J* = 8.9 Hz, H-4), 3.33-3.30 (m, H-2, overlapped with HDO), 3.07 (bd, 6H, *J* = 12.4 Hz, H-6), 2.85 (dd, 6H, *J* = 14.3 Hz, *J* = 7.0 Hz, H-6'), 2.75 - 2.65 (m, 12H, SCH₂), 2.08-1.95 (m, 12H, CH₂P), 1.23 (t, 36H, ³*J*_{H,H} = 7.0 Hz, OCH₂C*H*₃).

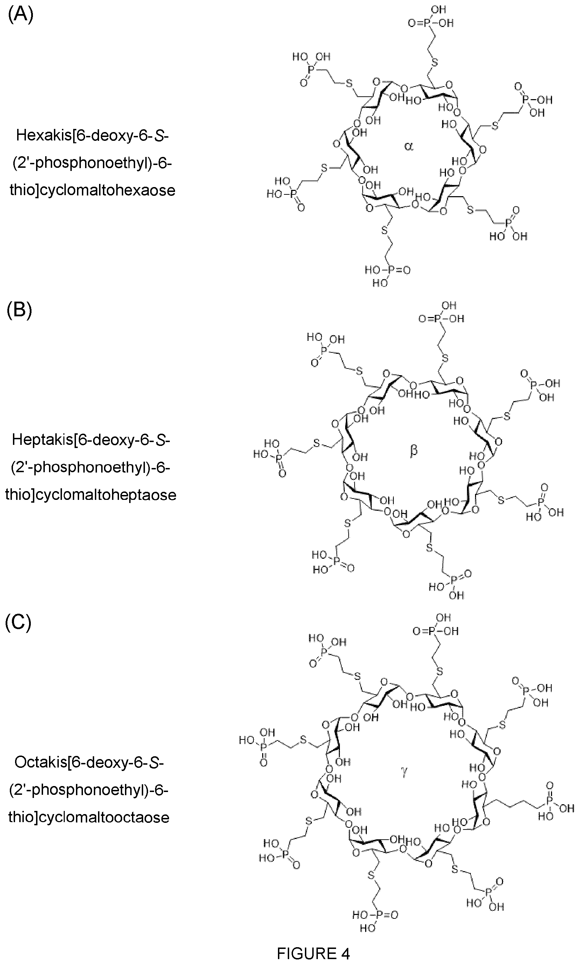

Preparation of product 4, hexakis[6-deoxy-6-*S*-(2'-phosphonoethyl)-6-thiolcyclomaltohexaose (Figure 4A): Trimethylsilyl bromide (203 µL, 1.538 mmol) was added dropwise to a solution of hexakis{6-deoxy-6-S-[2'-(diethoxyphosphoryl)ethyl]-6-thio}cyclomaltohexaose [product 3] (0.056 g, 0.025 mmol) in dry DMF (3 mL) at 0 °C under N₂ atmosphere. The mixture was stirred at room temperature overnight. Water (1 mL) was added and the solvent was evaporated under vacuum. The resulting residue was suspended in MeCN (55 mL), filtered, washed with MeCN (30 mL), dissolved in water (5 mL) and freeze-dried to yield heptakis[6-deoxy-6-S-(2'-phosphonoethyl)-6-thio]cyclomaltohexaose [product 4] (0.042 g, 0.024 mmol, 95 %) as a white solid. ¹H-NMR (600 MHz, D₂O), δ (ppm): 5.12 (bs, 6H, H-1), 4.01 (bs, 6H, H-5), 3.95 (t, 6H, *J* = 9.2 Hz, H-3), 3.65 (dd, 6H, ³*J*_{2,3} = 10.1 Hz, ³*J*_{1,2} = 3.3 Hz, H-2), 3.61 (t, 6H, *J* = 8.0 Hz, H-4), 3.28 (bd, 6H, *J* = 12.7 Hz, H-6), 3.00-2.97 (m, 6H, H-6'), 2.97-2.87 (m, 12H, CH₂S), 2.20-2.08 (m, 12H, CH₂P).

### Example 3: Synthesis of heptakis[6-deoxy-6-S-(2'-phosphonoethyl)-6-thiolcyclomaltoheptaose

This example describes the synthesis of heptakis[6-deoxy-6-S-(2'-phosphonoethyl)-6-thio]cyclomaltoheptaose, analogue of Subetadex with (2-phosphonoethyl)thio branches instead of (2-carboxyethyl)thio branches, based on a β-cyclodextrin core.

Preparation of reagent 5, heptakis(6-deoxy-6-iodo)cyclomaltoheptaose (Figure 2C): Heptakis(6-deoxy-6-iodo)cyclomaltoheptaose was prepared by the procedure described by Baer et al. ("Improved preparation of hexakis(6-deoxy)cyclomaltohexaose and heptakis(6-deoxy)cyclomaltoheptaose", Carbohydrate Research, 1992, 228, 307-314), by modifying the purification process to improve the purity of the obtained product. Briefly, heptakis(6-deoxy-6-iodo)cyclomaltoheptaose was prepared by the following procedure:

I₂ (4.7 g) was added in small portions to a stirred solution of triphenylphosphine (Ph₃P) (4.9 g) in dry *N,N*-dimethylformamide (DMF) (20 ml). After 30 min, β-cyclodextrin (1 g, dried under vacuum at 50 °C in the presence of P₂O₅ until constant weight) was also added. The mixture was stirred for 18 h at 80 °C under conditions preventing atmospheric moisture, then concentrated to half volume under reduced pressure, and cooled to 5 °C. It was then alkalinized with 3M NaOMe in MeOH to pH 9-10, and kept at room temperature for 30 min to allow formate esters solvolysis. The product was then precipitated by addition of MeOH (100 mL), filtered under vacuum, washed with MeOH (100 mL) and acetone (30 mL), and purified by Soxhlet extraction with MeOH. The resulting white solid was dried under vacuum at 60 °C until constant weight (1.1 g, 66%). The spectroscopic data of this material agreed with those described by Baer et al. (*supra*).

Preparation of product 6, heptakisf6-deoxy-6- S-r2'-(d iethoxyphosphoryl) ethyll-6-thiolcyclomaltoheptaose (Figure 3B): A solution of heptakis(6-deoxy-6-iodo)cyclomaltoheptaose [reagent 5] (0.3 g, 0.158 mmol), 2-(2-(diethoxyphosphoryl)ethyl)isothiouronium bromide [reagent 1] (0.693 g, 2.157 mmol) and cesium carbonate (1.081 g, 3.318 mmol) in dry DMF (10 mL) was stirred overnight. The solvent was then evaporated under vacuum and the residue was purified by column chromatography using acetonitrile-water (MeCN-H₂O 3:1) as eluent to afford heptakis{6-deoxy-6-S-[2'-(diethoxyphosphoryl)ethyl]-6-thio}cyclomaltoheptaose [product 6] (0.365 g, 0.152 mmol, 96 %) as a white solid. ¹H-NMR (600 MHz, DMSO-d₆), δ (ppm): 5.98 (d, 7H, ³*J* = 6.7 Hz, OH-2), 5.90 (d, 7H, 3J = 1.5 Hz, OH-2), 4.87 (d, 7H, ³*J*_{1,2} = 3.3 Hz, H-1), 4.04-3.94 (m, 28H, OC*H*₂CH₃), 3.78 (dt, 7H, *J* = 8.4 Hz, J = 2.1 Hz, H-5), 3.60 (t, 7H, *J* = 9.7 Hz, H-3), 3.41-3.33 (m, H-2,4, overlapped with HDO), 3.14 (bd, 7H, *J* = 12.6 Hz, H-6), 2.80 (dd, 7H, *J* = 14.7 Hz, *J* = 7.9 Hz, H-6'), 2.79-2.67 (m, 14H, SCH₂), 2.07-1.93 (m, 14H, CH₂P), 1.23 (dt, 42H, ³*J*_{H,H} = 7.3 Hz, ⁴*J*_{P,H} = 1.2 Hz, OCH₂C*H*₃).

Preparation of product 7, heptakis[6-deoxy-6-S-(2'-phosphonoethyl)-6-thiolcyclomaltoheptaose (Figure 4B): Trimethylsilyl bromide (390 µL, 2.940 mmol) was added dropwise to a solution of heptakis{6-deoxy-6-S-[2'-(diethoxyphosphoryl)ethyl]-6-thio}cyclomaltoheptaose [product 6] (0.1 g, 0.042 mmol) in dry DMF (5 mL) at 0 °C under N₂ atmosphere. The mixture was stirred at room temperature overnight. Water (1 mL) was added and the solvent was evaporated under vacuum. The resulting residue was suspended in MeCN (100 mL), filtered, washed with MeCN (50 mL), dissolved in water (5 mL) and freeze-dried to yield heptakis[6-deoxy-6-S-(2'-phosphonoethyl)-6-thio]cyclomaltoheptaose [product 7] (0.063 g, 0.031 mmol, 75 %) as a white solid. ¹H-NMR (300 MHz, D₂O), δ (ppm): 5.02 (d, 7H, ³*J*_{1,2} = 3.5 Hz, H-1), 3.95 (t, 7H, *J* = 7.8 Hz, H-5), 3.83 (t, 7H, *J* = 9.4 Hz, H-3), 3.55 (dd, 7H, ³*J*_{2,3} = 10.0 Hz, ³*J*_{1,2} = 3.7 Hz, H-2), 3.47 (t, 7H, *J* = 9.2 Hz, H-4), 3.16 (bd, *J* = 12.6 Hz, H-6), 2.92-2.74 (m, 21H, H-6', CH₂S), 2.06-1.95 (m, 14H, CH₂P).

### Example 4: Synthesis of octakis[6-deoxy-6-S-(2'-phosphonoethyl)-6-thiolcyclomaltooctaose

This example describes the synthesis of octakis[6-deoxy-6-S-(2'-phosphonoethyl)-6-thio]cyclomaltooctaose, analogue of Sugammadex with (2-phosphonoethyl)thio branches instead of (2-carboxyethyl)thio branches, based on a γ-cyclodextrin core.

Preparation of reagent 8, octakis(6-deoxy-6-iodo)cyclomaltooctaose (Figure 2D): Octakis(6-deoxy-6-iodo)cyclomaltooctaose was prepared by the procedure described by García-Fernández et al. ("Isothiocyanates and cyclic thiocarbamates of α,α'-trehalose, sucrose, and cyclomaltooligosaccharides", Carbohydrate Research, 1995, 268, 57-71), by modifying the purification process to improve the purity of the product obtained. Briefly, octakis(6-deoxy-6-iodo)cyclomaltooctaose was prepared by the following procedure:

I₂ (4.7 g) was added in small portions to a stirred solution of triphenylphosphine (Ph₃P) (4.9 g) in dry *N,N*-dimethylformamide (DMF) (20 ml). After 30 min, γ-cyclodextrin (1.2 g, dried under vacuum at 50 °C in the presence of P₂O₅ until constant weight) was also added. The mixture was stirred for 18 h at 80 °C under conditions preventing atmospheric moisture, then concentrated to half volume under reduced pressure, and cooled to 5 °C. It was then alkalinized with 3M NaOMe in MeOH to pH 9-10, and kept at room temperature for 30 min to allow formate esters solvolysis. The product was then precipitated by addition of MeOH (100 mL), filtered under vacuum, washed with MeOH (100 mL) and acetone (30 mL), and purified by Soxhlet extraction with MeOH. The resulting white solid was dried under vacuum at 60 °C until constant weight (1.2 g, 61%). The spectroscopic data of this material agreed with those described by García-Fernández et al. (*supra*).

Preparation of product 9, octakis{6-deoxy-6-*S*-[2'-(diethoxyphosphoryl)ethyl]-6-thiolcyclomaltooctaose (Figure 3C): A solution of octakis(6-deoxy-6-iodo)cyclomaltooctaose [reagent 8] (0.250 g, 0.115 mmol), 2-(2-(diethoxyphosphoryl)ethyl)isothiouronium bromide [reagent 1] (0.578 g, 1.800 mmol) and cesium carbonate (0.961 g, 2.949 mmol) in dry DMF (7.5 mL) was stirred overnight. The solvent was then evaporated under vacuum and the residue was purified by column chromatography using acetonitrile-water (MeCN-H₂O 10:3) as eluent to afford octakis{6-deoxy-6-S-[2'-(diethoxyphosphoryl)ethyl]-6-thio}cyclomaltooctaose [product 9] (0.252 g, 0.092 mmol, 80 %) as a white solid. ¹H NMR (600 MHz, DMSO-d₆) δ (ppm) 6.05 (s, 8H, OH-3), 5.99 (d, 8H, ³*J* = 5.9 Hz, OH-2), 4.93 (d, 8H, ³*J*_{1,2} = 3.4 Hz, H-1), 4.03-3.95 (m, 32H, OC*H*₂CH₃), 3.72 (dt, 8H, *J* = 8.4 Hz, *J* = 2.2 Hz, H-5), 3.57 (t, 8H, *J* = 9.3 Hz, H-3), 3.37-3.32 (m, H-2,4, overlapped with HDO), 3.16 (bd, 8H, *J* = 12.7 Hz, H-6), 2.79-2.67 (m, 24H, H-6', SCH₂), 2.08-1.95 (m, 16H, CH₂P), 1.24 (dt, 48H, ³*J*_{H,H} = 7.0 Hz, ⁴*J*_{P,H} = 1.5 Hz, OCH₂C*H*₃).

Preparation of product 10, octakis[6-deoxy-6-*S*-(2'-phosphonoethyl)-6-thiolcyclomaltooctaose (Figure 4C): Trimethylsilyl bromide (310 µL, 2.349 mmol) was added dropwise to a solution of octakis{6-deoxy-6-S-[2'-(diethoxyphosphoryl)ethyl]-6-thio}cyclomaltooctaose [product 9] (0.080 g, 0.029 mmol) in dry DMF (3 mL) at 0 °C under N₂ atmosphere. The mixture was stirred at room temperature overnight. Water (1 mL) was added and the solvent was evaporated under vacuum. The resulting residue was suspended in MeCN (60 mL), filtered, washed with MeCN (30 mL), dissolved in water (5 mL) and freeze-dried to afford octakis[6-deoxy-6-S-(2'-phosphonoethyl)-6-thio]cyclomaltooctaose [product 10] (0.051 g, 0.022 mmol, 76 %) as a white solid. ¹H-NMR (600 MHz, D₂O), δ (ppm): 5.12 (d, 8H, ³*J*_{1,2} = 3.8 Hz, H-1), 4.03 (dt, 8H, *J* = 8.6 Hz, *J* = 2.2 Hz, H-5), 3.90 (t, 8H, *J* = 9.5 Hz, H-3), 3.66 (dd, 8H, ³*J*_{2,3} = 9.9 Hz, ³*J*_{1,2} = 3.8 Hz, H-2), 3.57 (t, 8H, *J* = 9.3 Hz, H-4), 3.23 (bd, 8H, *J* = 12.0 Hz, H-6), 2.98 (dd, 8H, *J* = 13.8 Hz, *J* = 7.9 Hz, H-6'), 2.94-2.85 (m, 16H, CH₂S), 2.21-2.09 (m, 16H, CH₂P).

### Example 5: Interaction of octakis[6-deoxy-6-S-(2'-phosphonoethyl)-6-thio]cyclomaltooctaose (product 10) with rocuronium bromide

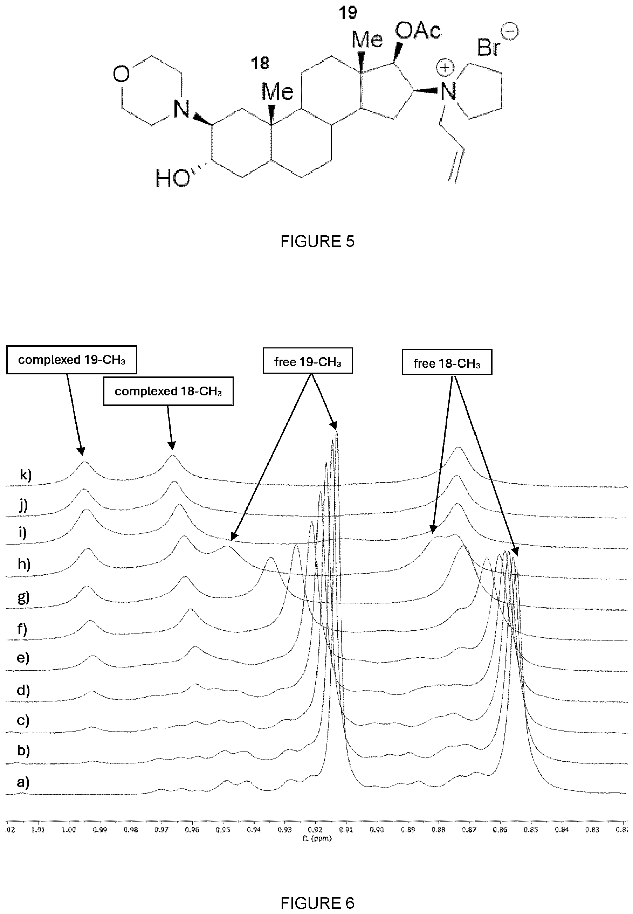

Rocuronium bromide (Figure 5) is an aminosteroid drug used in anaesthesia that acts on the motor end-plate, competing for cholinergic receptors. Rocuronium CAS number is 143558-00-3. Sugammadex is an oligosaccharide of the cyclodextrin family used to block the effect of the neuroblocking anaesthetic drugs rocuronium and vecuronium, so that the use of Sugammadex promotes the recovery of muscle tone after surgery.

In the literature, the interaction of Sugammadex (Bridion^{®}) with rocuronium has been studied by nuclear magnetic resonance (NMR) in D₂O at pH 7.5 through the signals corresponding to the 18-CH₃ and 19-CH₃ methyl groups of rocuronium (Kenneth S. Cameron et al., "An NMR study of cyclodextrin complexes of the steroidal neuromuscular blocker drug rocuronium bromide", Magnetic Resonance in Chemistry, 2002, 40, 251-260). Since the complexation process is slow on the time scale of the NMR experiment, the signals of the 18-CH₃ and 19-CH₃ groups of the free rocuronium molecule are located at δ 0.86 and 0.92 ppm, respectively, while the signals of the 18-CH₃ and 19-CH₃ groups of the complexed rocuronium molecule are located at δ 0.97 and 0.95 ppm, respectively. The progress of the titration process produces changes in the intensity of each of these signals, so that the intensity of the 18-CH₃ and 19-CH₃ signals of the free molecule is maximal in the absence of Sugammadex and decreases with the progress of the titration, while the signals of the 18-CH₃ and 19-CH₃ groups of the complexed molecule only appear in the presence of Sugammadex and increase with the progress of the titration.

In the present invention, the interaction of octakis[6-deoxy-6-S-(2'-phosphonoethyl)-6-thio]cyclomaltooctaose (product 10) with rocuronium bromide was studied by a nuclear magnetic resonance (NMR) titration experiment in which a solution of 1 mM rocuronium bromide in 20 mM phosphate buffer, pH 7. 2, in D₂O was titrated with increasing volumes of a solution of 1.5 mM product 10 in 20 mM phosphate buffer, pH 7.2, in D₂O. The results were similar to those described in the literature for Sugammadex (Figure 6).

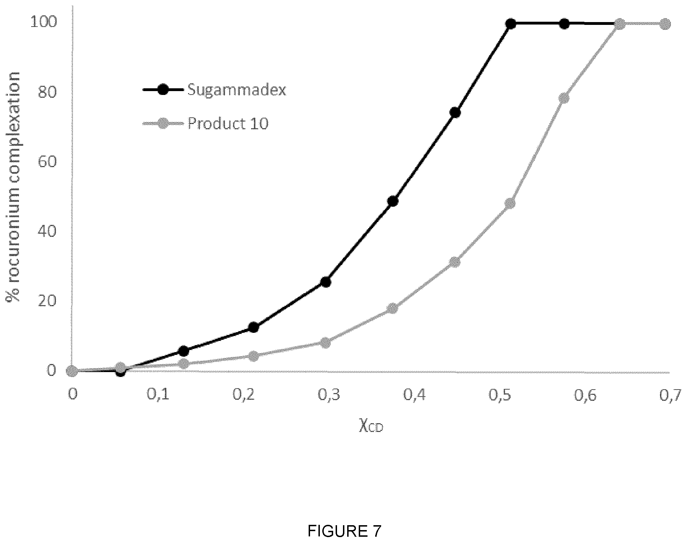

In particular, the intensity ratio between the 19-CH₃ signals of complexed rocuronium and free rocuronium allows the calculation of the percentage of rocuronium complexation for each concentration ratio. The graphical plot of this percentage versus the mole fraction of product 10 of each of these mixtures can be seen in Figure 7. The results obtained for the rocuronium-Sugammadex pair has also been represented in that figure for comparative purposes.

From the data observed in Figure 7, it can be inferred that:
- The binding constant of rocuronium bromide by octakis[6-deoxy-6-S-(2'-phosphonoethyl)-6-thio]cyclomaltooctaose (product 10) is in the same order of magnitude as that for Sugammadex (10⁷ M⁻¹). (A. Bom et al., "A novel concept of reversing neuromuscular block: chemical encapsulation of rocuronium bromide by a cyclodextrin-based synthetic host", Angewandte Chemie International Edition, 2002, 41, 266-270).
- The complex stoichiometry of rocuronium bromide with octakis[6-deoxy-6-S-(2'-phosphonoethyl)-6-thio]cyclomaltooctaose (product 10) is 1:2 (rocuronium bromide-product 10), while that for rocuronium bromide complex with Sugammadex is 1:1.

## Claims

1. An α-, β-, or γ-cyclodextrin derivative, wherein the hydroxyl groups at all C-6 positions of the α-, β-, or γ-cyclodextrin are replaced with 2-mercaptoethylphosphonic acid residues as [2-(diethoxyphosphoryl)ethyl]thio branches according to formula 1: 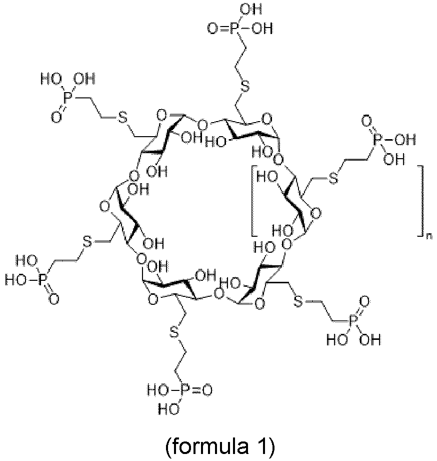 wherein n = 6-8.

2. The α-, β-, or γ-cyclodextrin derivative according to claim 1, wherein the derivative is hexakis[6-deoxy-6-S-(2'-phosphonoethyl)-6-thio]cyclomaltohexaose, heptakis[6-deoxy-6-S-(2'-phosphonoethyl)-6-thio]cyclomaltoheptaose, or octakis[6-deoxy-6-S-(2'-phosphonoethyl)-6-thio]cyclomaltooctaose.

3. A method for obtaining an α-, β-, or γ-cyclodextrin derivative according to any one of claims 1 or 2, wherein the method comprises:
a) preparing α-, β-, or γ-cyclodextrin derivatives where the hydroxyl groups at all C-6 positions of the α-, β-, or γ-cyclodextrin are replaced with iodine atoms;
b) replacing the iodine atoms at all C-6 positions of the α-, β-, or γ-cyclodextrin with [2-(diethoxyphosphoryl)ethyl]thio branches by using the isothiouronium salt of diethyl 2-mercaptoethylphosphonate; and
c) hydrolysing the diethyl esters of the [2-(diethoxyphosphoryl)ethyl]thio branches to give phosphonic acids as (2-phosphonoethyl)thio branches (-S-CH₂-CH₂-PO(OH)₂).

4. An α-, β-, or γ-cyclodextrin derivative according to any one of claims 1 or 2 for use in medicine.

5. An α-, β-, or γ-cyclodextrin derivative according to any one of claims 1 or 2 for use in the treatment or prevention of lysosomal diseases.

6. An α-, β-, or γ-cyclodextrin derivative according to any one of claims 1 or 2 for use in the antagonistic treatment of the anaesthetics rocuronium and vecuronium.

7. Use of an α-, β-, or γ-cyclodextrin derivative according to any one of claims 1 or 2 as an ampicillin carrier, or as an anticancer drug carrier.

8. Use of an α-, β-, or γ-cyclodextrin derivative according to any one of claims 1 or 2 as packing material for HPLC columns, preferably in the form of Cu complexes, or as a chiral selector in capillary electrophoresis.
